# EUROPEAN PATENT SPECIFICATION

(11) **EP 2 722 394 B1**
(45) Date of publication and mention of the grant of the patent: **21.03.2018**
(21) Application number: 11867262.5
(22) Date of filing: 07.06.2011
(51) Int. Cl.: C12P 19/26, C12P 19/18, C07H 13/04

(54) **OBTAINING OLIGOSACCHARIDES BY MEANS OF A BIOTECHNOLOGICAL PROCESS**
GEWINNUNG VON OLIGOSACCHARIDEN MITTELS EINES BIOTECHNOLOGISCHEN VERFAHRENS
OBTENTION D'OLIGOSACCHARIDES PAR UN PROCÉDÉ BIOTECHNOLOGIQUE

(43) Date of publication of application: 23.04.2014
(73) Proprietor: Hero AG, 5600 Lenzburg (CH)
(72) Inventor: MATENCIO HILLA, Esther, E-30008 Murcia (ES); NAVARRO BARRERA, Verónica, E-46370 Valencia (ES); ENRIQUE LÓPEZ, María, E-12590 Castellón (ES); TORTAJADA SERRA, Marta, E-46015 Valencia (ES); LUKOVIC, Dunja, E-41940 Sevilla (ES); RODRÍGUEZ JIMENEZ, Diego, E-46014 Valencia (ES); RAMÓN VIDAL, Daniel, E-46183 Valencia (ES); ÁLVAREZ PÉREZ, Beatriz, E-46001 Valencia (ES)
(74) Representative: Carlos Hernando, Borja
(86) International application number: PCT/ES2011/070403
(87) International publication number: WO 2012/168495

(56) References cited:
- US-A1- 2007 275 881
- US-A1- 2007 275 881
- US-A1- 2010 019 648
- MURATA T ET AL: "FACILE ENZYMATIC CONVERSION OF LACTOSE INTO LACTO-N-TETRAOSE AND LACTO-N-NEOTETRAOSE", GLYCOCONJUGATE JOURNAL, CHAPMAN & HALL, BOSTON, vol. 16, no. 3, 1 March 1999 (1999-03-01), pages 189-195, XP008029474, ISSN: 0282-0080, DOI: 10.1023/A:1007020219275
- DATABASE WPI Week 199950 December 1999 (1999-12) Thomson Scientific, London, GB; AN 1999-583703 XP055139135, & JP 11 253191 A (MEIJI MILK PROD CO LTD) 21 September 1999 (1999-09-21)
- PRIEM BERNARD ET AL: "A new fermentation process allows large-scale production of human milk oligosaccharides by metabolically engineered bacteria", GLYCOBIOLOGY, OXFORD UNIVERSITY PRESS, US, vol. 12, no. 4, 1 April 2002 (2002-04-01), pages 235-240, XP002380632, ISSN: 0959-6658, DOI: 10.1093/GLYCOB/12.4.235
- MATSUO ICHIRO ET AL: "Cloning and overexpression of beta-N-acetylglucosaminidase encoding gene nagA from Aspergillus oryzae and enzyme-catalyzed synthesis of human milk oligosaccharide", BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, vol. 67, no. 3, 1 March 2003 (2003-03-01), pages 646-650, XP009130482, ISSN: 0916-8451, DOI: 10.1271/BBB.67.646 [retrieved on 2003-06-28]
- PERA LICIA M ET AL: "Purification and characterization of a thermostable and highly specific beta-N-acetyl-D-glucosaminidase from Aspergillus niger 419", BIOTECHNOLOGY AND APPLIED BIOCHEMISTRY, ACADEMIC PRESS, US, vol. 26, no. 3, 1 December 1997 (1997-12-01), pages 183-187, XP008164156, ISSN: 0885-4513
- DATABASE EMBL [Online] 28 January 2007 (2007-01-28), "Aspergillus niger contig An09c0050, genomic contig", XP002735202, retrieved from EBI accession no. EM_STD:AM270194 Database accession no. AM270194 & PEL HERMAN J ET AL: "Genome sequencing and analysis of the versatile cell factory Aspergillus niger CBS 513.88.", NATURE BIOTECHNOLOGY, vol. 25, no. 2, February 2007 (2007-02), pages 221-231, ISSN: 1087-0156
- SCHWAB C ET AL: "Heterologous expression of glycoside hydrolase family 2 and 42 beta-galactosidases of lactic acid bacteria in Lactococcus lactis", SYSTEMATIC AND APPLIED MICROBIOLOGY, URBAN & FISCHER, AMSTERDAM, NL, vol. 33, no. 6, 1 October 2010 (2010-10-01), pages 300-307, XP027298410, ISSN: 0723-2020 [retrieved on 2010-09-06] -& DATABASE EMBL [Online] 2 July 2009 (2009-07-02), "Streptococcus thermophilus lacZ gene for beta-galactosidase, strain FUA3194", XP002735203, retrieved from EBI accession no. EM_STD:FN424354 Database accession no. FN424354
- YOON J-H ET AL: "The synthesis of galactopyranosyl derivatives with beta-galactosidases of different origins", CARBOHYDRATE RESEARCH, PERGAMON, GB, vol. 292, no. 1, 4 October 1996 (1996-10-04), pages 153-163, XP004018964, ISSN: 0008-6215, DOI: 10.1016/0008-6215(96)00187-5
- MATSUO, I. ET AL.: 'Cloning and overexpression of beta-N-acetylglucosaminidase encoding gene nagA from Aspergillus oryzae and enzyme-catalyzed synthesis of human milk oligosaccharide' BIOSCIENCE BIOTECHNOLOGY AND BIOCHEMISTRY vol. 67, no. 3, March 2003, pages 646 - 650, XP009130482
- MURATA, T. ET AL.: 'Facile enzymatic conversion of lactose into lacto-N-tetraose and lacto-N-neotetraose.' GLYCOCONJUGATE JOURNAL. vol. 16, no. 3, March 1999, pages 189 - 195, XP008029474
- MIYAZAKI, T. ET AL.: 'Enzymatic synthesis of lacto-N-difucohexaose I which binds to Helicobacter pylori.' METHODS IN ENZYMOLOGY vol. 480, 2010, pages 511 - 524, XP008164122
- DATABASE WPI Week 199950, 06 February 2012 Derwent Publications Ltd., London, GB; AN 1999-583703, XP055139135 & JP 11 253191 A (MEIJI MILK PROD CO LTD) 21 September 1999

<110> HERO ESPAÑA S.A.
<120> OBTAINING OLIGOSACCHARIDES BY MEANS OF A BIOTECHNOLOGICAL PROCESS
   <130> AX130354EP
   <140> PCT/ES2011/070403
   <141> 2011-06-07
   <160> 6
<170> BISSAP
Sequence
   <210> 1
   <211> 1815
   <212> DNA
   <213> Aspergillus niger
   <400> 1 
atgctccttg ctcgcctctg gccggcgtgc ctcacgctgc tcgtggccgg ggccgccgcc 60

gttaaggtca atcctttacc agctccgcgc aacatttctt ggacttcttc gggtcctaag 120

cagctggcaa gctttgtcag tctccgtgca tcgcaagata cccctgactt cattctcgcc 180

aatggatgga atcgcgcatg ggattctatt gtctccttac aatgggtacc cgcagcgacc 240

gaaggcccca tgtcatccta tcagcctttt cccaccgcga cggccgcggc cggggtcact 300

aaacggtcct cgcaggcact gccatcgctg cagtttgttg atgtgaatgt ggctgatctt 360

gacgcggatc tgcagcatgg agttgacgag tcttacacac tggaggtcac cgagagcgct 420

acttcagtcg tcatcgaggc acccactgtc tggggagcac tccatgcctt tactactctc 480

cagcagctgg tcatctctga cggacagggc ggcttgataa tcgagcagtc agttaagatc 540

caggatgcgc cgctctatcc ttaccggggt atcatgcttg atactggtcg aaattttatc 600

tcagttagca agatctatga gcagctggac ggcatgtcgc tttctaagct caatgttcta 660

cactggcaca tggaagacac ccaatcgtgg ccagtgcaga ttgatgccta tccagagatg 720

atccatgatg cctactcccc gcgcgaggta tattcacacg ccgatatgcg caacatcgta 780

gcgtatgccc gagcacgcgg cgtacgggtc attcctgaga ttgatatgcc cagccattcc 840

gcttctggat ggaaacaggt tgacccccag atggtcacct gcgtggattc ctggtggtct 900

aatgatgatt atgccttgca taccgcggta gaacccccac ctggtcaaat ggacatcatt 960

tacaacggca catatgatgt ggtccgagag gtatataatg agctgtccgg tatcttcccg 1020

gacaactggt tccatgttgg agcggatgag attcagccta actgctttaa cttcagcagc 1080

tacgtcaccc agtggttcgc agaggatccg tcgcgcactt acaatgatct ggcccagtac 1140

tgggtagacc atgctgtacc gatcttccag aactacagtt cctcgcggca gctggttatg 1200

tgggaagata tcgtgctatc tacggaacat gcgcatgatg tacccacaaa tatcgtgatg 1260

caaacctgga acaacgggct ggattacatc aatcagctga ctgccaaagg ctacgatgtg 1320
 
attgtgtcat catcggactt catgtatctg gactgtggga tgggtggctt cctaaccaat 1380

gatccccgat acgatgtaat gagcaatcca gatccaaaca ctcccaactt caactatggc 1440

ggcaacggag gctcgtggtg tgcgccgtac aagacgtggc agcgtatcta cgactatgac 1500

ttcacgcaaa atctgactga cgcccaggcc cagcacatcg tgggcgcgga ggcacccctc 1560

tggtcggaac aagtagatga tgtgactgtc tccagtctgt tctggccgcg cgccgctgct 1620

ctggcagagc tggtatggtc tgggaatcgg gatgagaatg gacataagcg cacgacgctg 1680

atgacgcagc gtattttgaa ctttcgcgag tacttggtgg ccaatggggt gcaggcgaaa 1740

gcgctggttc ctaaatactg tgtgcagcgc ccacatacct gcgacttgta tcgcaaccag 1800

agtgtaattc agtaa 1815
Sequence
   <210> 2
   <211> 3081
   <212> DNA
   <213> Streptococcus thermophilus
   <400> 2 
atgaacatga ctgaaaaaat tcaaacttat ttaaacgatc caaagattgt tagcgttaat 60

actgttgatg ctcactcaga tcataagtat tttgaatctc ttgaagaatt ttctgaaggg 120

gagatgaagt taagacaatc tcttaatgga aaatggaaaa ttcactatgc tcagaataca 180

aatcaggttt taaaagactt ttataaaaca gaatttgatg aaactgattt gaatttcatc 240

aatgtaccag gtcatttaga gcttcaaggt tttggttctc cacaatatgt gaatacccaa 300

tatccttggg atggtaaaga attccttcgt ccacctcaag ttcctcaaga atcaaatgct 360

gttgcatcat acgttaaaca ttttaccttg aatgatgcat taaaagataa aaaagtattt 420

atctcattcc aaggggttgc tacttccatc tttgtatggg tcaatggtaa cttcgtagga 480

tacagtgaag attcatttac acctagtgaa tttgaaatta gtgattacct tgttgaaggt 540

gataacaagt tggcggtagc tgtttatcgt tactctacag caagctggtt ggaagaccaa 600

gacttctgga gactttacgg tatttttaga gatgtttact tgtatgctat tccaaaagtt 660

cacgttcaag atctctttgt taagggagat tatgattacc aaacaaaagc aggtcaattg 720

gatattgatt tgaagactgt tggtgattat gaagacaaga agattaaata tgttctttca 780

gattatgaag gcatcgttac agaaggtgat gcatctgtta atggtgacgg tgaactatct 840

gtaagtcttg aaaatcttaa aatcaaacct tggagtgctg aaagtcctaa actttacgat 900

ttgatccttc atgttttgga tgatgaccaa gttgttgaag tcgttccagt taaagttgga 960

tttagacgct ttgaaattaa agataaactt atgcttttga atggtaagag aattgtcttt 1020
 
aaaggggtta acagacacga atttaacgct agaacaggac gttgtatcac tgaagaagat 1080

atgctttggg atatcaaagt gatgaaacaa cataacatca atgctgttcg tacttcacac 1140

tatcctaacc aaacacgttg gtatgaattg tgtgatgaat atggacttta tgttatcgat 1200

gaagccaacc ttgaaacaca cggtacatgg caaaaacttg gtctatgcga accttcatgg 1260

aatatcccag ctagtgaacc agaatggttg cctgcttgtt tggatcgtgc caataacatg 1320

ttccaacgcg ataagaacca cgctagtgtt atcatttggt cttgtggtaa tgaatcatat 1380

gctggtaaag atattgctga catggctgat tacttccgta gtgttgacaa tactcgtcca 1440

gttcactatg aaggtgttgc atggtgtcgt gagtttgatt acattacaga catcgaaagt 1500

cgtatgtatg cgaaaccagc tgatatcgaa gaatacctca caactggtaa actagttgat 1560

ctttcaagcg ttagtgataa acactttgct tcaggtaacc taactaacaa acctcaaaaa 1620

ccttatattt catgtgaata catgcacaca atgggtaact ctggtggtgg attgcaactc 1680

tacactgact tagagaaata tccagaatac caaggtggat ttatttggga cttcattgac 1740

caagctattt acaaaacact tccaaatggt agcgaattcc tatcatatgg tggtgactgg 1800

catgatagac cttctgacta cgaattttgt ggaaatggta tcgtctttgc agatcgtacc 1860

ctaactccaa aacttcaaac agttaaacat ctttactcta atattaagat tgctgttgat 1920

gaaaaatcag taactatcaa gaatgataat ctcttcgaag atctttctgc ttatactttc 1980

ctagctagag tttacgaaga tggtagaaaa gttagtgaaa gtgaatatca ctttgatgtt 2040

aaaccaggcg aagaagcaac attcccagtt aactttgtag tcgaggcttc aaattctgaa 2100

caaatttacg aagttgcttg tgttctgagg gaagcaactg aatgggctcc taaaggtcat 2160

gaaattgttc gtggtcaata tgttgttgaa aagattagca ctgaaacacc agttaaagca 2220

cctttgaatg ttgttgaagg cgacttcaac atcggtattc aaggacaaaa cttctcaatc 2280

ttgctttcac gtgcacaaaa tactttagta tctgctaagt ataatggtgt tgaattcatt 2340

gagaaaggtc ctaaacttag cttcactcgt gcttacactg acaacgatcg tggtgctgga 2400

tatccattcg aaatggcagg ctggaaggtt gctggaaact atagtaaagt tacagatact 2460

caaattcaaa tcgaagacga ctctgttaaa gtgacttatg ttcatgaatt gccaggcttg 2520

tctgatgtcg aagttaaggt aacttatcaa gttgattaca agggtcgaat ctttgttact 2580

gcaaactatg atggtaaagc aggtttgcca aacttccctg aatttggtct agaatttgct 2640

atcggttcac aatttacaaa ccttagctat tatggatacg gtgcagaaga aagctaccgt 2700

gataaacttc ctggtgccta tcttggtcga tatgaaacat ctgttgaaaa gacatttgct 2760

ccatatctaa tgccacaaga atctggtaat cactatggta ctcgtgaatt cacagtatct 2820

gatgataacc ataatggtct taaattcacc gcacttaata aagcattcga attcagtgct 2880

ttgcgtaaca gtactgaaca aattgaaaat gctcgtcacc aatatgagtt gcaagaatct 2940
 
gatgctacat ggattaaagt tcttgctgct caaatgggtg taggtggtga cgacacatgg 3000

ggtgctccag ttcatgacga attcttgctt agctcagcag atagctatca attaagcttc 3060

atgattgaac cactaaatta g 3081
Sequence
   <210> 3
   <211> 91
   <212> Type : PRT
   <213> Artificial Sequence
   <400> 3 
MRFPSIFTAV LFAASSALAA PVNTTTEDET AQIPAEAVIG YSDLEGDFDV AVLPFSNSTN 60
NGLLFINTTI ASIAAKEEGV SLEKREAEAA G 91
Sequence
   <210> 4
   <211> Length : 21
   <212> Type : PRT
   <213> Artificial Sequence
   <400> 4
   MLLQAFLFLL AGFAAKISAL Q 21
Sequence
   <210> 5
   <211> 19
   <212> PRT
   <213> Artificial Sequence
   <400> 5
MFKSVVYSIL AASLANALQ 19
Sequence
   <210> 6
   <211> 602
   <212> PRT
   <213> Artificial Sequence
   <400> 6

MAYFRLYAVL LAVASSVAAV KVNPLPAPRH ISWGHSGPKP LSDVSLRTER DTDDSILTNA 60

WNRAWETIVS LEWVPAGIEA PIPEFDEFPT STPSASAAAT RSKRANVPIQ FVDVDVEDWD 120

ALQHGVDESY TLDAKAGSDA IDITAKTVWG ALHAFTTLQQ LVISDGNGGL ILEQPVHIKD 180

APLYPYRGLM VDTGRNFISV RKLHEQLDGM ALSKLNVLHW HLDDTQSWPV HIDAYPEMTK 240

DAYSARETYS HDDLRNVVAY ARARGIRVIP EIDMPAHSAS GWQQVDPDIV ACANSWWSND 300

NWPLHTAVQP NPGQLDIINP KTYEVVQDVY EELSSIFTDD WFHVGGDEIQ PNCYNFSTYV 360

TEWFQEDPSR TYNDLMQHWV DKAVPIFRSV SDSRRLVMWE DVVLNTEHAD DVPTDIVMQS 420

WNNGLENINK LTERGYDVIV SSADFMYLDC GRGGYVTNDD RYNEQTNPDP DTPSFNYGGI 480

GGSWCGPYKT WQRIYNYDFT LNLTNAQAKH VIGATAPLWS EQVDDVNISN LFWPRAAALA 540

ELVWSGNRDA KGNKRTTLFT QRILNFREYL LANGVMAATV VPKYCLQHPH ACDLNYDQTV 600

LH 602

## Description

### Technical Field of the Invention

The present invention relates to obtaining specific oligosaccharides present in breast milk, specifically lacto-N-neotetraose and lacto-N-tetraose, by means of a biotechnological process.

### Background of the Invention

Oligosaccharides are short chain carbohydrates (degree of polymerization of 2 to 7) naturally occurring in fruits, vegetables, human milk and honey. They are considered important due to their biological and physiological properties, as functional ingredients and particularly due to their prebiotic activity.

The oligosaccharides present in human milk play an important biological role since they are probably related to infection prevention in newborns by preventing the adhesion of certain pathogens (viruses and bacteria) to the intestinal epithelium, triggering the immune system locally and acting as prebiotics.

Breast milk contains more than 130 different oligosaccharides making it the third most abundant component. The total concentration thereof decreases as breastfeeding progresses such that it contains less than half the amount of oligosaccharides after one year than in the early weeks of life. There are quantitative and qualitative differences between the oligosaccharides detected in the milk of different mammals. The most abundant monomers of human milk oligosaccharides are D-glucose, D-galactose, N-acetyl glucosamine, L-fucose and N-acetyl neuraminic acid. All these monomers undergo modifications through the addition of L-fucose and/or sialic acid groups, generating the so-called neutral or acidic oligosaccharides, respectively, between 150 and 200 of which have been described. The concentration thereof in milk decreases as breastfeeding progresses, however they seem to have a relevant physiological role in the early months.

The most important neutral oligosaccharides are formed from lactose by means of glucosyltransferases (GT), such that the most abundant neutral oligosaccharides formed in human milk are lacto-N-triose and lacto-N-tetraose and the different isomers of lacto-N-fucopentaose. The non-digested oligosaccharide fraction in breast milk stimulates bifidobacteria growth in the colon, and this flora may have beneficial effects for protection against enteric infections. Therefore, oligosaccharides are a primary component of the innate immune system through which a mother protects her child from pathogens (enteric pathogens or pathogens affecting another location) during breastfeeding.

It is noteworthy that none of these products is found in goat milk, sheep milk or cow milk. Furthermore, these molecules are not present in artificial milks, so adding them to artificial milks has a clear social and economic interest. In this sense, there would be four possibilities for their production and subsequent use.
i) The first possibility would be to purify them from human milk. This alternative is unapproachable given the lack of raw material.
ii) The second possibility would be chemical synthesis. Even though this is possible, the cost thereof is high and the efficiency low.
iii) The third alternative is based on using enzymes to produce different oligosaccharides from lactose. This strategy is based on using different glycosidases or glycosyltransferases from various origins (microorganisms or mammals). The use of glycosyltransferases is preferred given their greater efficiency, however they are too costly since many of them originate from mammals. Therefore, work is being done in expressing genes encoding one of these enzymes in different microorganisms (*Escherichia coli, Pichia pastoris* and *Saccharomyces cerevisiae*) for the purpose of overproducing them, although this approach has never reached the semi-industrial scale.
iv) Finally, the last strategy includes constructing microorganisms capable of generating the oligosaccharides by means of genetic engineering techniques. In this sense, initial progress has been made by expressing three genes of *Neisseria meningitiditis* encoding an N-acetyl glucosamine transferase, a galactosyl transferase and a sialyl transferase in a strain of *E*. *coli* in which the expression of the lactose transporter-encoding gene can be induced by glycerol. Although this approach has been successful, marketing the obtained product for baby food seems to be complex since pathogenic bacteria are used as gene donors and receivers and the obtained ingredients are generated from genetically modified organisms which would require specific labeling. Different transgenic bacteria which, when grown in a fermenter and permeabilized, are each capable of producing different enzymatic activities and precursors involved in the synthesis of the oligosaccharides of interest have also been successfully constructed. Said approach involves four different microorganisms making the possible industrial application thereof difficult.

With respect to the state of the art described in patent documents, there are earlier processes, such as JP 11253191 A for example, which provides a method for enzymatically synthesizing lacto-N-tetraose, suitable for use in foods and medicinal products in any amount. The method comprises reacting UDP-GlcNac on lactose in the presence of beta-1,3-N-acetylglucosaminyl transferase for synthesizing lacto-N-triose followed by binding with galactose in the presence of beta-galactosidase, lacto-N-tetraose thus being obtained.

Document US 2007/0275881 A1 relates to pharmaceutical compositions and the use thereof in the treatment of infectious diseases. The compositions contain peptides bound to oligosaccharides of interest, such as lacto-N-tetraose and lacto-N-neotetraose. It describes obtaining these oligosaccharides in yeasts in the presence of the corresponding glycosyltransferases.

The invention of document US 7,521,212 B1 relates to the production of oligopolysaccharides through a microbiological process in which lactose-N-neotetraose is obtained, for example, in the presence of lactose and GlcNAc and the enzymes beta-1-3-N-acetylglucosaminyltransferase and beta-1-4-galactosyltransferase. It also describes obtaining the intermediate product lacto-N-triose.

Document US 6,204,431 B1 relates to methods for producing transgenic non-human mammals capable of producing certain oligosaccharides in their milk. For the purpose of the invention described in the mentioned document, the genetic sequences of enzymes such as beta-acetylglucosaminyltransferase from eukaryotic or prokaryotic species are selected and inserted into the pronucleus of the embryo which will lead to obtaining the transgenic animal.

Document ES 2 162 619 T3 describes an apparatus and a method for obtaining oligosaccharide compositions in three steps by using human glucosaminyltransferases and galactosyltransferases, and document US 5,879,912 describes a method for synthesizing oligosaccharides, polysaccharides, glycolipids and glycoproteins. It describes the synthesis of lacto-N-neotetraose in the presence of two cell cultures providing two enzymes with glycosyltransferase activity to form the beta-Gal 1-4 GlcNAc and beta-GlcNAc 1-3 bonds in the presence of UPD-GlcNAc and lactose.

### Object of the Invention

The present invention relates to the development of a biotechnological production method for producing specific oligosaccharides present in breast milk, specifically for producing the oligosaccharides lacto-N-neotetraose and lacto-N-tetraose from lacto-N-triose obtained from culture supernatants rich in the enzymes suitable for generating them from their precursors: lactose and N-acetyl-glucosamine.

### Description of the Drawings

Figure 1 shows the enzymatic formation of the oligosaccharides: lacto-N-triose, lacto-N-neotetraose and lacto-N-tetraose.
Figure 2 shows the plate assay for detecting N-acetyl-glucosaminidase activity (Example 3).
Figure 3 shows the effect of temperature and culture medium on the activity of *A. niger* nagA enzyme recovered from the culture broth of the recombinant strain of *S*. *cerevisiae* (Example 4).
Figure 4 shows the determination of beta-galactosidase activity in culture broth (A) or cell suspension (B) of *S*. *cerevisiae* or *P. pastoris* recombinants for the *S*. *thermophilus* lacZ enzyme (Example 5).
Figure 5 shows the subcellular localization of *S*. *thermophilus* lacZ overexpressed in *S*. *cerevisiae* (Example 6).
Figure 6 shows the plate assay for detecting beta-galactosidase activity in *E*. *coli* transformants for the *S*. *thermophilus lacZ* gene (Example 7).
Figure 7 shows the process of obtaining lacto-N-triose in culture broth (Example 8).
Figure 8 shows the influence of substrate concentration on the lacto-N-triose production in culture broth (Example 9).
Figure 9 shows the process of obtaining lacto-N-tetraose and lacto-N-neotetraose in two sequential steps (Example 10).

### Detailed Description of the Invention

The method of the invention is defined in the appended claims 1-9.

That is, the invention concerns a method for producing lacto-N-triose and/or lacto-N-tetraose and/or lacto-N-neotetraose which comprises carrying out a reaction using at least one culture of genetically modified yeasts which provide the activities of the enzymes responsible for producing lacto-N-triose and/or lacto-N-tetraose and/or lacto-N-neotetraose from lactose, which comprises:
a) adding lactose and N-acetylglucosamine in the presence of beta-(1,3)-N-acetylglucosaminyltransferase to form lacto-N-triose, and
b) adding galactose in the presence of beta-galactosyltransferase to form lacto-N-tetraose and/ or lacto-N-neotetraose,
c) allowing lacto-N-triose and/or lacto-N-tetraose and/or lacto-N-neotetraose to accumulate, and
d) recovering lacto-N-triose and/or lacto-N-tetraose and/or lacto-N-neotetraose from said reaction mixture,
   characterized in that
   the enzymes necessary for producing the lacto-N-triose, and/or lacto-N-tetraose and/or lacto-N-neotetraose are produced by genetically modified yeasts from the species S*.* cerevisiae, which carry a recombinant vector comprising the nagA gene of Aspergillus niger SEQ 10 NO: 1 expressing an enzyme with beta-1-3-N-acetylglucosaminyltransferase activity and a recombinant vector comprising the lacZ gene of Streptococcus thermophilus SEQ 10 NO: 2 expressing an enzyme with beta-galactosyltransferase activity.

The oligosaccharides to be obtained in the present invention are the tetrasaccharides lacto-N-neotetraose and lacto-N-tetraose. These tetrasaccharides are present in breast milk and are formed by one unit of lactose to which N-acetylglucosamine (beta 1-3 bond) is added generating lacto-N-triose. Lacto-N-neotetraose (beta 1-4 bond) and lacto-N-tetraose (beta 1-3 bond) can be obtained from lacto-N-triose and in the presence of beta-galactosyltransferase. The tetrasaccharides are therefore produced by means of two sequential activities, a beta-(1,3)-N-acetylglucosaminyltransferase activity, and then a beta-(1,3) or beta-(1,4) galactosyltransferase activity.

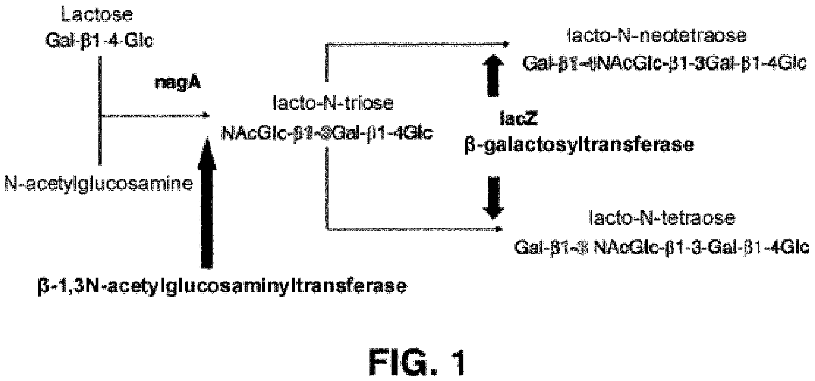

The method follows the simplified scheme of enzymatic transglycosylation reactions necessary for the synthesis of the oligosaccharides of interest, i.e., lacto-N-neotetraose and lacto-N-tetraose, as well as lacto-N-triose as an intermediate product (Figure 1).

The enzymes necessary for producing the oligosaccharides lacto-N-triose, lacto-N-tetraose and lacto-N-neotetraose are produced and secreted into the culture broth by genetically modified yeasts of the species *S*. *cerevisiae.* In order to use a microorganism which produces enzymes for human consumption, a series of legal requirements focusing on the obligation for such microorganism to be listed in the so-called "GRAS" (generally acknowledged as secure) list must be met, i.e., the microorganism meets certain safety requirements. There are about 50 approved GRAS microorganisms for the food industry. In the present invention, the genes encoding both enzymatic activities originate from GRAS microorganisms to prevent rejection problems. The selected genes are: *Aspergillus niger nagA* gene and *Streptococcus thermophilus lacZ* gene.

The enzymatic activity necessary for the first step of the process has been demonstrated to be present in bovine serum, colostrum, and the enzymes encoded by the *nagA* genes of the fungi *Aspergillus oryzae* and *Aspergillus nidulans* (two enzymes with N-acetyl-glucosaminidase activity).

Due to similarity with the species in which glucosaminyltransferase-encoding genes have been identified (Matsuo 2003), the public availability of its sequence and its GRAS status, the fungus *A. niger* is selected as the origin of this enzymatic activity to be used in the process object of the invention. It must be indicated that even though this activity has been detected in the species *A. niger,* the corresponding gene has not been identified in its genomic sequence. Therefore, for the purpose of the present invention, it was necessary to first identify the gene in *A. niger* which is orthologous to the *A. nidulans nagA* gene. The nucleotide sequence of the *A. niger nagA* gene is shown in SEQ ID NO:1.

The second step is carried out by a galactosyltransferase enzyme. This activity has been demonstrated in some bacterial and fungal beta-galactosidases (*A. oryzae, Bifidobacterium bifidum, Bacillus circulans).*

In this case, the gene encoding the beta-galactosidase (lacZ) enzyme of the bacterium *S*. *thermophilus* has been selected as the origin of the galactosyltransferase activity to be used in the process object of the invention because said gene encodes this activity in other lactic bacteria, and *S*. *thermophilus* is known to be capable of catalyzing transglycosylation reactions by acting on lactose (Smart 1991, Reuter *et al.* 1999). The *lacZ* gene is identified in the genomic sequence of *S*. *thermophilus* and is illustrated in SEQ ID NO:2.

On the other hand, for *S*. *cerevisiae* to secrete the cloned enzymes into the culture broth, it is necessary to fuse them with a secretion signal peptide. The three most widely studied secretion signal peptides for *S*. *cerevisiae* are α factor secretion signal peptide (Brake *et al.* 1989), SEQ ID NO:3, SUC2 invertase secretion signal peptide (Perlman *et al.* 1982), SEQ ID NO:4, and PHO5 acid phosphatase secretion signal peptide (Arima *et al.* 1983), SEQ ID NO:5.

Therefore, the genes encoding the enzymes nagA and lacZ can be expressed in *S*. *cerevisiae* transformant which can be obtained by inserting the DNA into two vectors and introducing said vectors in a host. The early methods of *S*. *cerevisiae* transformation involved cell wall removal for producing spheroplasts which may incorporate DNA after treating the spheroplasts with calcium and polyethylene glycol (Hinnen *et al.* 1978). The transformants were plated in isotonic selective medium for cell wall regeneration. A more practical method was later developed in which intact cells are made competent by treatment with lithium ions (Ito *et al.* 1983). This method is used today despite the fact that it is less efficient than the preceding one; however, a modification using DMSO allows up to a 25-fold increase in transformation frequency. A third method, electroporation, has been used more recently, very high efficiencies being obtained (Meilhoc *et al.* 1990).

The vectors to be used for this purpose can be any of those capable of expressing the genes encoding the enzymes in suitable hosts. Said vectors can for example include: plasmid, bacteriophage or cosmid vectors. Said vectors will generally comprise a regulating factor such as a promoter; they must have a region at a short distance from the promoter which supplies the entry site for the ribosome upon being transcribed; and finally they must contain an origin of replication allowing their multiplication in the host. Furthermore, the vectors selected for each protein have different selection marker genes for the purpose of rescuing different auxotrophies, which allows the simultaneous introduction of both vectors in one and the same host and selection in a single step. The most widely used selection markers for *S*. *cerevisiae* are LEU2, TRP1, URA3 and HIS3, used in the corresponding mutant strains which are auxotrophic for leucine, tryptophan, uracil and histidine, respectively (Beggs 1978; Rose *et al.* 1984; Struhl *et al.* 1980; Tschumper *et al.* 1980). Such auxotrophic strains are available and their selection requires the use of minimum growth medium in the absence of the relevant nutrient. The following type culture collections can be mentioned as sources for obtaining said strains: Yeast Genetics Stock Culture Center of the American Type Culture Collection (http://www.atcc.org/SearchCatalogs/YeastGeneticStock.cfm.), EUROSCARF (http://www.uni-frankfurt.de/fb15/mikro/esuoscarf/col_index.html), Research Genetics (http://www.resgen/products/YEASTD.php3), National Collection of Yeast Cultures (http://www.ncyc.co.uk/Sacchgen.html.), Centraalbureau voor Schimmelcultures (http://www.cbs.knaw.nl/yeast/WebC.ASP) and Culture Collection of *Saccharomyces cerevisiae* (http://www.natur.cuni.cz/fccm/federacs.htm#dgub.).

The following can be mentioned as hosts in which an expression vector containing the DNA be introduced: yeasts, bacteria and cell cultures of insect, mammal, etc. The use of the yeast *S*. *cerevisiae* is preferred, but *Schizosaccharomyces pombe, P. pastoris, E. coli, Bacillus subtilis, S. thermophilus,* etc., are also proposed.

To check the expression of the *nagA* gene in the host, it is possible to assay the hydrolytic N-acetylglucosaminidase activity of the enzyme expressed in the obtained transformants by means of plate assay or in liquid medium, using the synthetic substrates methylumbelliferyl (MU)-N-acetylglucosamine or p-nitrophenyl (PNP)-N-acetylglucosamine, respectively (Borooah *et al.* 1961). In the case of plate assay, the formation of methylumbelliferone as a result of the hydrolytic N-acetyl-glucosaminidase activity is qualitatively detected by means of fluorescence analysis under UV light. Methylumbelliferone is a fluorogenic compound which is fluorescent when illuminated with a UV lamp of 366 nm. The liquid assay allows quantifying the activity by analyzing the absorbance at 405 nm due to the release of p-nitrophenol, a colored compound which absorbs at a wavelength of 405 nm in an alkaline solution, as a result of the hydrolytic N-acetyl-glucosaminidase activity.

Similarly, to check the expression of the *lacZ* gene it is possible to assay the hydrolytic beta-galactosidase activity of the enzyme expressed in the obtained transformants by means of plate assay or in liquid medium with the synthetic substrates methylumbelliferyl (MU)-galactoside or p-nitrophenyl (PNP)-galactopyranoside, respectively. (Maruhn 1976). In the case of the plate assay, the formation of methylumbelliferone as a result of the hydrolytic beta-galactosidase activity is qualitatively detected by means of fluorescence analysis under UV light. Methylumbelliferone is a fluorogenic compound which is fluorescent when illuminated with a UV lamp of 366 nm. The liquid assay allows quantifying the activity by analyzing the absorbance at 405 nm due to the release of p-nitrophenol, a colored compound which absorbs at a wavelength of 405 nm in an alkaline solution, as a result of the hydrolytic beta-galactosidase activity.

The plate assay allows a fast selection of those clones with higher gene expression. On the other hand, the liquid assay allows quantifying the activity as well as the secretion since the activity in the culture broth and in the isolated cells is independently analyzed.

Once the activity of the selected recombinant enzymes is checked, it is possible to express them simultaneously in one and the same strain using plasmids which rescue different auxotrophies. The plasmids to be used can be those belonging to the collection described in Mumberg *et al.* (1995), formed by series of expression vectors with the combinations of four promoters, two origins of replication and four different markers. By using these vectors, it is possible to control gene expression at different levels, controlling the transcription level as well as the copy number of the recombinant gene.

Once the transformants with greater activity of each enzyme or with simultaneous activities in one and the same strain are selected, it is possible to grow them to a high density obtaining culture broths with high enzymatic activities or large amounts of cells with intracellular activity in the event that the enzyme is not secreted. These culture broths or cell extracts can be used directly as a reaction bed for obtaining the oligosaccharides of interest from their precursors lactose and N-acetyl-glucosamine without the need for purifying the corresponding proteins. Alternatively, the enzymes can be purified for use in obtaining the oligosaccharides of interest.

Therefore, the final process of obtaining the oligosaccharides of interest will consists of two phases, a first phase which will last for 70 to 74 hours at 43-47°C, preferably, 72 hours at 45°C, in which lacto-N-triose will be generated from its precursors lactose and N-acetyl-glucosamine using the nagA activity of culture broths or cell extracts (in the event that the protein is or is not secreted, respectively) or purified protein. When this phase ends, it is possible to purify the lacto-N-triose or to continue the process with a second phase to generate lacto-N-tetraose and lacto-N-neotetraose. This second phase will last for 36 hours at 45°C in which lacto-N-tetraose and lacto-N-neotetraose will be generated from lacto-N-triose and galactose, present in the reaction broth resulting from the first phase, using the lacZ activity of a culture broth or cell extract, depending on whether or not the recombinant enzyme is secreted, or where appropriate, purified protein. Preferably, the method for obtaining lacto-N-tetraose and lacto-N-neotetraose has been carried out such that the lacto-N-tetraose and lacto-N-neotetraose are obtained in a range of 1 to 100 grams per liter of reaction broth in two sequential steps from culture broth rich in nagA activity and cell extract with lacZ activity, originating from a single strain expressing and secreting nagA and expressing and not secreting lacZ.

For the purpose of the present invention, the possibility of carrying out the reaction using the activity of the two enzymes simultaneously present in one and the same culture broth or in one and the same cell extract with lacZ activity, originating from a single strain expressing and secreting nagA and lacZ, is also contemplated.

Finally, the oligosaccharides obtained according to the present invention are extracted and purified from the reaction medium through common techniques known in the state of the art. Chromatographic separation on charcoal (Whistler *et al.* 1950) is used as the preferred oligosaccharide purification technique.

Once purified, these oligosaccharides can be incorporated as food ingredients in food products for consumption.

### Examples

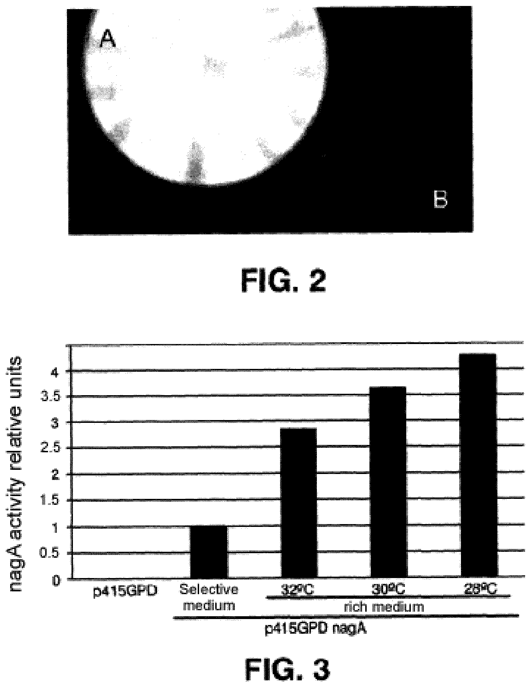

### Example 1: Nucleotide sequence of A. niger nagA gene (Figure 2)

The gene encoding the N-acetyl-glucosaminidase activity in *A. niger* is identified by homology with the *A. nidulans nagA* gene. The sequence of said reference gene is available and annotated in the databases (http://www.broad.mit.edu/annotation/genome/aspergillus_group) (http://www.ncbi.nlm.nih.gov/entrez/viewer/fcgi?db=protein&id=10039359). The protein resulting from the expression thereof corresponds with SEQ ID NO:6.

By comparing the preceding amino acid sequence with the *A. niger* genome available in the databases (http://genome.igi-psi.org/Asoni1/Asoni1.home.html), there are at least 2 possible genes which are orthologous to *A. nidulans nagA* gene. Of the two, the gene the sequence of which is SEQ ID NO:1 is cloned due to its greater homology and its description in the databases as a gene encoding a putative N-acetyl-glucosaminidase activity. The alignment of the amino acid sequences encoded by both genes shows a homology of 71.3%. The cloning was performed by means of polymerase chain reaction (PCR) from the genomic DNA of *A. niger* following the method and materials described in Mullis *et al.* (1986).

### Example 2: Nucleotide sequence of S. thermophilus lacZ gene

The sequence of the *lacZ* gene encoding the *S*. *thermophilus* β-galactosidase enzyme is identified and available in the databases and it corresponds with SEQ ID NO:2. The protein is described as a tetramer of about 124 kDa. The *lacZ* gene was cloned by means of polymerase chain reaction (PCR) from the genomic DNA of *S*. *thermophilus* following the method and materials described in Mullis *et al.* (1986).

### Example 3: Plate determination of the N-acetyl-glucosaminidase activity of A. niger nagA gene overexpressed in S. cerevisiae

The transformants generated with plasmid p415GPD-nagA-LEU2 in the haploid strain BY4741 (MATa HIS3D1 LEU2D0 MET15D0 URA3D0) are grown on a YPD rich medium formulated with 10 g/l of yeast extract (Scharlau Chemie S.A.), 20 g/l of bacteriological peptone (Pronadisa) and 20 g/l of glucose (Panreac Química S.A.U.) as a carbon source, solidified with 2% agar (Pronadisa).

A liquified solution of the substrate methylumbelliferyl (MU)-N-acetyl-glucosamine at a concentration of 1 mM in 45 mM citrate buffer with 1% agarose at pH 4.8 is poured on the plate, incubated at 50°C for 30 minutes protected from light and observed under UV light. The N-acetyl-glucosaminidase activity causes the formation of methylumberylferone which is a fluorogenic compound that is fluorescent when illuminated with a UV lamp of 366 nm.

As shown in Figure 2, a fluorescent halo is observed around those clones capable of secreting the nagA enzyme (A) with respect to control strains that do not express nagA (B).

### Example 4: Effect of temperature and culture medium on the activity of A. niger nagA enzyme recovered from the culture broth of the recombinant strain of S. cerevisiae

For the purpose of optimizing the expression of *nagA* gene in *S*. *cerevisiae,* a study on whether the composition of the medium and/or the growth temperature can influence the enzyme expression and/or secretion was conducted.

To that end, a recombinant strain generated by means of transforming the haploid strain BY4741 (MATa HIS3D1 LEU2D0 MET15D0 URA3D0) with the plasmid p415GPD-nagA-LEU2 was used. The transformants were selected in synthetic SD medium based on 6.7 g/l of yeast nitrogen base without amino acids (Difco) and supplemented with 20 g/l of glucose (Panreac Química S.A.U.) as a carbon source and 200 mg/l of histidine (Merck), uracil (Calbiochem) and methionine (Fluka) as nutritional requirements.

First, the cells were cultured in parallel in a minimum selective medium and in a rich medium. Synthetic SD medium based on 6.7 g/l of yeast nitrogen base without amino acids (Difco) and supplemented with 20 g/l of glucose (Panreac Química S.A.U.) as a carbon source and 200 mg/l of histidine (Merck), uracil (Calbiochem) and methionine (Fluka) as nutritional requirements was used as the minimum medium; YPD formulated with 10 g/l of yeast extract (Scharlau Chemie S.A.), 20 g/l of bacteriological peptone (Pronadisa) and 20 g/l of glucose (Panreac Química S.A.U.) as a carbon source was used as the rich medium.

The culture broth resulting from growing the strain in both media was assayed for N-acetyl-glucosaminidase activity in liquid medium using p-nitrophenyl (PNP)-N-acetyl-glucosamine as the reaction substrate. To that end, an aliquot of each culture broth was used and diluted in 0.09 M citrate/phosphate buffer at pH 4.8 in the presence of the substrate, incubating at 30°C for 30 minutes. The hydrolytic N-acetyl-glucosaminidase activity was quantified by means of determining the absorbance at 405 nm due to the release of p-nitrophenol, a colored compound which absorbs at a wavelength of 405 nm in an alkaline solution, as a result of same.

As observed in Figure 3, the activity is almost three times greater in the culture supernatant when the strain is grown in rich medium than in minimum selective medium, clearly showing the influence of culture medium on nagA enzyme expression and/or secretion.

In terms of the growth temperature, the selected transformant was grown in parallel in a rich YPD medium formulated with 10 g/l of yeast extract (Scharlau Chemie S.A.), 20 g/l of bacteriological peptone (Pronadisa) and 20 g/l of glucose (Panreac Química S.A.U.) as a carbon source at 3 different temperatures: 28, 30 and 32°C. The culture broth was analyzed in terms of its hydrolytic N-acetyl-glucosaminidase activity in liquid medium. To that end, an aliquot of the culture broth was used and diluted in 0.09 M citrate/phosphate buffer at pH 4.8 in the presence of the substrate p-nitrophenyl (PNP)-N-acetyl-glucosamine, incubating at 30°C for 30 minutes. The hydrolytic N-acetyl-glucosaminidase activity was quantified by means of determining the absorbance at 405 nm due to the release of p-nitrophenol, a colored compound which absorbs at a wavelength of 405 nm in an alkaline solution, as a result of same.

As shown in Figure 3, the activity recovered in the culture supernatant gradually increases as the growth temperature drops, being 50% greater when the growth temperature drops from 32 to 28°C. Therefore, an optimal growth temperature of between 26 and 30°C is established for nagA expression and secretion.

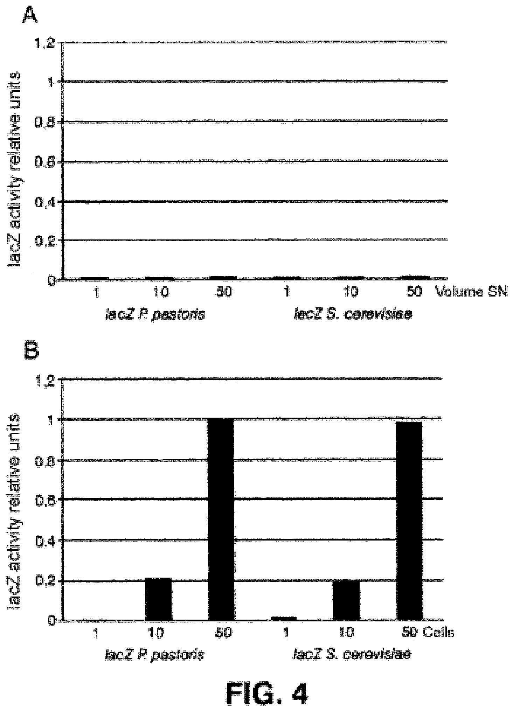

As shown in Figure 4, up to 3 times more N-acetyl-glucosaminidase activity is recovered in the culture broth when the nagA transformant grows in rich medium and more than 4 times more activity is recovered when the growth temperature further drops to 28°C.

### Example 5: Determination of β-galactosidase activity of S. thermophilus lacZ gene overexpressed in the yeasts S. cerevisiae and P. pastoris

Recombinant strains of *S*. *cerevisiae* and *P. pastoris* were generated by transforming the haploid strain BY4741 (MATa HIS3D1 LEU2D0 MET15D0 URA3D0) with the plasmid p416GPD-lacZ-URA3 and the wild-type haploid strain X-33 with the plasmid pPICzaB-lacZ, respectively. The *S*. *cerevisiae* transformants were selected in synthetic SD medium based on 6.7 g/l of yeast nitrogen base without amino acids (Difco) and supplemented with 20 g/l of glucose (Panreac Química S.A.U.) as a carbon source and 200 mg/l of histidine (Merck), leucine (Merck) and methionine (Fluka) as nutritional requirements. The *P. pastoris* transformants were selected in YPDS medium formulated with 10 g/l of yeast extract (Scharlau Chemie S.A.), 20 g/l of bacteriological peptone (Pronadisa), 20 g/l of glucose (Panreac Química S.A.U.) as a carbon source, 1 M of sorbitol (Applichem VWR) and 100 µg/ml of Zeocin (Invivogen).

They were grown in the indicated media and the hydrolytic beta-galactosidase activity in the culture broths as well as in a cell suspension was determined. To that end, the indicated amount of broth or of cell suspension was diluted in 100 mM phosphate buffer at pH 7 in the presence of the substrate p-nitrophenyl (PNP)-galactopyranoside, incubating at 55°C for 30 minutes. The hydrolytic beta-galactosidase activity was quantified by means of determining the absorbance at 405 nm due to the release of p-nitrophenol, a colored compound which absorbs at a wavelength of 405 nm in an alkaline solution, as a result of same.

As shown in Figure 4, no beta-galactosidase activity is detected in the supernatant culture. It is also not detected in the case of *P. pastoris* recombinant or in the case of *S*. *cerevisiae* (A). The levels of activity in the cell suspensions are similar in the two organisms (B).

### Example 6: Subcellular localization of lacZ in S. thermophilus lacZ recombinants in S. cerevisiae

The recombinant strain of *S*. *cerevisiae* was generated by means of transforming the haploid strain BY4741 (MATa HIS3D1 LEU2D0 MET15D0 URA3D0) with the plasmid p416GPD-lacZ-URA3 and selecting in synthetic SD medium based on 6.7 g/l of yeast nitrogen base without amino acids (Difco) and supplemented with 20 g/l of glucose (Panreac Química S.A.U.) as a carbon source and 200 mg/l of histidine (Merck), leucine (Merck) and methionine (Fluka) as nutritional requirements.

The recombinant was grown in rich YPD medium formulated with 10 g/l of yeast extract (Scharlau Chemie S.A.), 20 g/l of bacteriological peptone (Pronadisa) and 20 g/l of glucose (Panreac Química S.A.U.) as a carbon source at 28°C. Three samples with the same number of cells were separated: a suspension of whole cells was prepared, one of them for analyzing activity in the cell wall; protoplasts were prepared with another one of the samples by means of treating the cells with 3 mg/ml of zymolyase 20T (Seikagaku) for 15 minutes at 37°C for evaluating the lacZ activity in the plasma membrane; and finally, the third sample was mechanically lysed and a cell extract was prepared in which the cytoplasmic lacZ activity was evaluated. The three samples were prepared in the same volume.

Using a portion of each of the samples, an assay for determining the hydrolytic beta-galactosidase activity in liquid medium was carried out by diluting each sample in 100 mM phosphate buffer at pH 7 in the presence of the substrate p-nitrophenyl (PNP)-galactopyranoside and incubating at 55°C for 30 minutes. The hydrolytic beta-galactosidase activity was quantified by means of determining the absorbance at 405 nm due to the release of p-nitrophenol, a colored compound which absorbs at a wavelength of 405 nm in an alkaline solution, as a result of same.

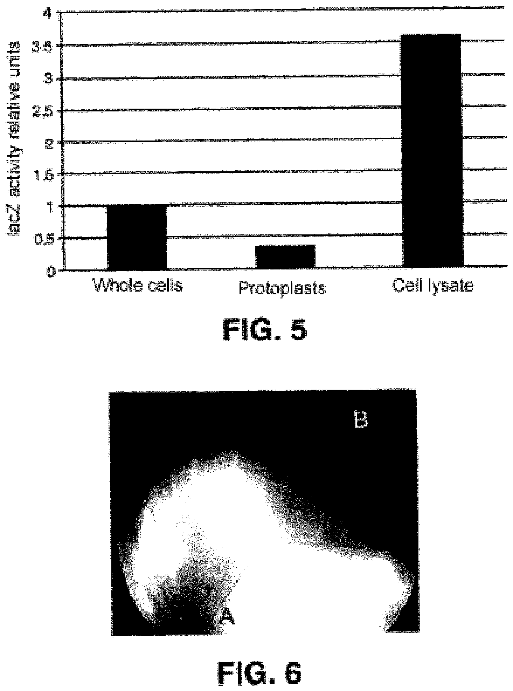

As observed in Figure 5, 3 times more activity is recovered in the cell lysate than in the cell wall. The activity in the cell membrane determined from the prepared protoplasts is very little. Therefore, the lacZ activity in this recombinant is mainly found in the cell cytoplasm and a small portion is retained in the cell wall.

### Example 7: Plate determination of the beta-galactosidase activity of S. thermophilus lacZ gene overexpressed in E. coli

An *E*. *coli* transformant for the *S*. *thermophilus lacZ* gene is generated by means of transforming the strain DH5α (*fhuA2* Δ*(argF-lacZ)U169 phoA glnV44 √80 Δ(lacZ)M15 gyrA96 recA1 relA1 endA1 thi-1 hsdR17*) with the plasmid p416GPD-lacZ URA3 and selecting in LB medium formulated with 5 g/l of yeast extract (Scharlau Chemie S.A.), 10 g/l of tryptone (Scharlau Chemie S.A.), 10 g/l of sodium chloride (NaCl) and ampicillin (Calbiochem) at a concentration of 100 mg/l.

A liquefied solution of the substrate methylumbelliferyl (MU)-galactopyranoside at a concentration of 1 mM in 100 mM phosphate buffer with 1% agarose at pH 6 is poured on the plate, incubated at 50°C for 30 minutes protected from light and observed under UV light. The beta-galactosidase activity causes the formation of methylumbelliferone which is a fluorogenic compound that is fluorescent when illuminated with a UV lamp of 366 nm.

As shown in Figure 6, a fluorescent halo is observed around those clones capable of secreting the lacZ enzyme (A) with respect to control strains that do not express lacZ (B).

### Example 8: Obtaining lacto-N-triose from culture broth rich in nagA activity and from its precursors: lactose and N-acetyl-glucosamine

To carry out the reaction for obtaining lacto-N-triose, a recombinant strain generated by means of transforming the haploid strain BY4741 (MATa HIS3D1 LEU2D0 MET15D0 URA3D0) with the plasmid p415GPD-nagA-LEU2 was used. The transformants were selected in synthetic SD medium based on 6.7 g/l of yeast nitrogen base without amino acids (Difco) and supplemented with 20 g/l of glucose (Panreac Química S.A.U.) as a carbon source and 200 mg/l of histidine (Merck), uracil (Calbiochem) and methionine (Fluka) as nutritional requirements.

The selected transformant was grown to OD 40 in batches in 4xYPD medium formulated with 40 g/l of yeast extract (Scharlau Chemie S.A.), 80 g/l of bacteriological peptone (Pronadisa) and 80 g/l of glucose (Panreac Química S.A.U.) as a carbon source at 28°C until saturation.

The presence of the nagA enzyme in the culture supernatant was checked by means of determining the hydrolytic N-acetyl-glucosaminidase activity in an aliquot of the culture broth diluted in 0.09 M citrate/phosphate buffer at pH 4.8 in the presence of the substrate, incubating at 30°C for 30 minutes. The hydrolytic N-acetyl-glucosaminidase activity was quantified by means of determining the absorbance at 405 nm due to the release of p-nitrophenol, a colored compound which absorbs at a wavelength of 405 nm in an alkaline solution, as a result of same.

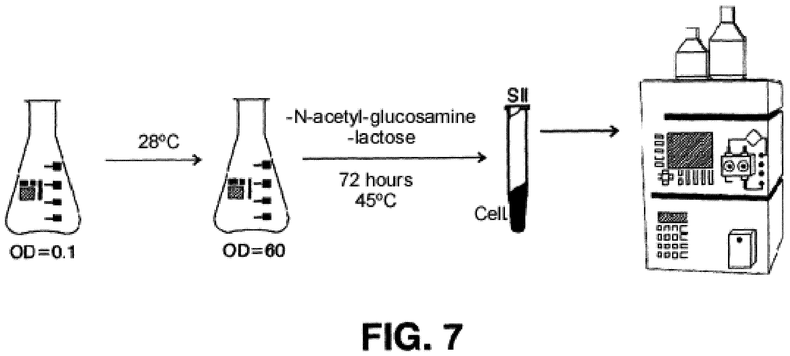

As schematized in Figure 7, lactose and N-acetyl-glucosamine were then subsequently added to the culture medium directly at a high concentration (330 g/l and 100 g/l respectively) and incubated at 45°C for 72 hours. A fraction of the supernatant was filtered and the presence of lacto-N-triose therein was analyzed by means of HPLC.

### Example 9: Influence of substrate concentration on lacto-N-triose production in culture broth

For the purpose of optimizing the lacto-N-triose production process, assays such as those described above were carried out with decreasing amounts of the substrates lactose and N-acetyl-glucosamine. The reaction carried out with 416 g/l of lactose and 125 g/l of N-acetyl-glucosamine was used as the reference and reactions with 50%, 33%, 25%, 10% and 1% of the substrates were carried out in parallel.

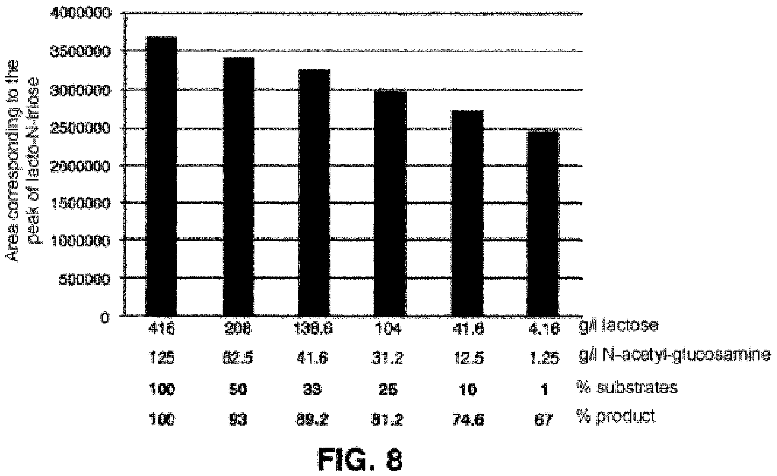

As observed in Figure 8, the yield of the reaction decreases as the substrate concentration in the medium decreases. However, this trend is not proportional, only 7% of the product being lost for a reduction of up to 50% in substrate concentration and 33% with a reduction of up to 99%.

### Example 10: Obtaining lacto-N-tetraose and lacto-N-neotetraose in two sequential steps from culture broth rich in nagA activity and cell extract with lacZ activity, originating from a single strain expressing and secreting nagA and expressing and not secreting lacZ

The haploid strain BY4741 (MATa HIS3D1 LEU2D0 MET15D0 URA3D0) was transformed with the plasmids p415GPD-nagA-LEU2 and p416GPD-lacZ-URA4 simultaneously. The transformants were selected in synthetic SD medium based on 6.7 g/l of yeast nitrogen base without amino acids (Difco) and supplemented with 20 g/l of glucose (Panreac Química S.A.U.) as a carbon source and 200 mg/l of histidine (Merck) and methionine (Fluka) as nutritional requirements. The obtained strain, recombinant for both enzymes, only secretes the *nagA* protein. This allows separating the two activities in one and the same culture; the nagA activity is enriched in the culture broth and the lacZ activity inside the cell.

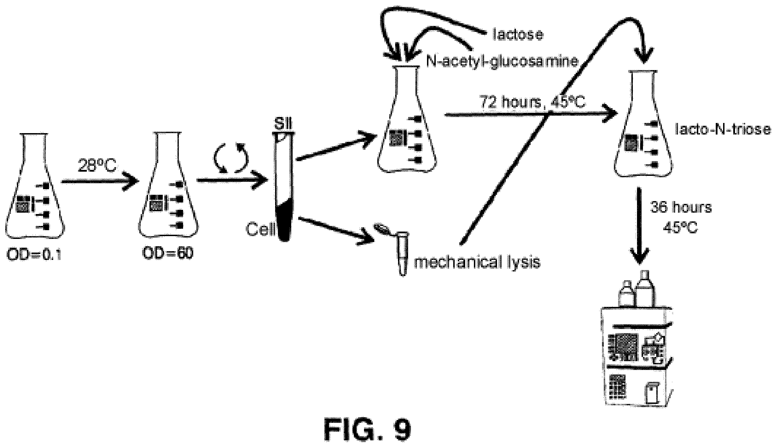

As shown in Figure 9, the strain is grown to a high density in 4xYPD medium formulated with 40 g/l of yeast extract (Scharlau Chemie S.A.), 80 g/l of bacteriological peptone (Pronadisa) and 80 g/l of glucose (Panreac Química S.A.U.) as a carbon source at 28°C until saturation. The cells were then separated from the culture supernatant. Lactose and N-acetyl-glucosamine at a concentration of 330 g/L and 100 g/L, respectively, were added to the latter and incubated at 45°C for 72 hours. The cells were then lysed and a cell extract which is added to the reaction was prepared, being incubated for another 36 hours at the same temperature. A fraction of the reaction broth was filtered and the presence of lacto-N-tetraose and lacto-N-neotetraose therein was analyzed by means of HPLC. The amount of lacto-N-tetraose and lacto-N-neotetraose was determined in 3.5 grams per liter of reaction broth.

### References

- Arima K, Oshima T, Kubota I, Nakamura N, Mizunaga T, Toh-e A. The nucleotide sequence of the yeast PHO5 gene: a putative precursor of repressible acid phosphatase contains a signal peptide. Nucleic Acids Res. 1983 Mar 25;11(6):1657-72.
- Beggs JD. Transformation of yeast by a replicating hybrid plasmid. Nature. 1978 Sep 14;275(5676):104-9.
- Borooah J, Leaback DH, Walker PG. Studies on glucosaminidase. 2. Substrates for N-acetyl-beta-glucosaminidase. Biochem J. 1961 Jan;78(1):106-10.
- Brake AJ. Secretion of heterologous proteins directed by the yeast alpha-factor leader. Biotechnology. 1989;13:269-80.
- Hinnen A, Hicks JB, Fink GR. Transformation of yeast. Proc Natl Acad Sci USA. 1978 Apr;75(4):1929-33.
- Ito H, Fukuda Y, Murata K, Kimura A. Transformation of intact yeast cells treated with alkali cations. J Bacteriol. 1983 Jan;153(1):163-8.
- Maruhn D. Rapid colorimetric assay of beta-galactosidase and N-acetyl-beta-glucosaminidase in human urine. Clin Chim Acta. 1976 Dec;73(3):453-61.
- Matsuo I, Kim S, Yamamoto Y, Ajisaka K, Maruyama JI, Nakajima H, Kitamoto K. Cloning and overexpression of beta-N-acetylglucosaminidase encoding gene nagA from Aspergillus oryzae and enzyme-catalyzed synthesis of human milk oligosaccharide. Biosci Biotechnol Biochem. 2003 Mar;67(3):646-50.
- Meilhoc E, Masson JM, Teissié J. High efficiency transformation of intact yeast cells by electric field pulses. Biotechnology (NY). 1990 Mar;8(3):223-7.
- Mumberg D, Müller R, Funk M. Yeast vectors for the controlled expression of heterologous proteins in different genetic backgrounds. Gene. 1995 Apr; 156(1):1 19-22.
- Mullis K, Faloona F, Scharf S, Saiki R, Horn G, Erlich H. Specific enzymatic amplification of DNA in vitro: the polymerase chain reaction. Cold Spring Harb Symp Quant Biol. 1986 51 Pt 1:263-73.
- Perlman D, Halvorson HO, Cannon LE. Presecretory and cytoplasmic invertase polypeptides encoded by distinct mRNAs derived from the same structural gene differ by a signal sequence. Proc Natl Acad Sci USA. 1982 Feb;79(3):781-5.
- Reuter S, Rusborg A, Zimmermann W. β-Galactooligosaccharide synthesis with β-galactosidases from Sulfolobus solfataricus, Aspergillus oryzae, and Escherichia coli. Enzyme and Microbial Technology. 1999 Sep;25 (6):509-16.
- Rose M, Grisafi P, Botstein D. Structure and function of the yeast URA3 gene: expression in Escherichia coli. Gene. 1984 Jul-Aug;29(1-2):113-24.
- Smart JB. Transferase reactions of the β-galactosidase from Streptococcus thermophilus. Applied Microbiology and Biotechnology 1991 34(4):495-501.
- Struhl K, Davis RW. A physical, genetic and transcriptional map of the cloned his3 gene region of Saccharomyces cerevisiae. J Mol Biol. 1980 Jan 25;136(3):309-32.
- Tschumper G, Carbon J. Sequence of a yeast DNA fragment containing a chromosomal replicator and the TRPI gene. Gene. 1980 Jul; 10(2):157-66.
- Whistler RL, Durso DF. Chromatographic separation of sugars on charcoal. Journal of the American Chemical Society 1950 Feb;72:677-679.
- Mullis K, Faloona F, Scharf S, Saiki R, Horn G, Erlich H., "Specific enzymatic amplification of DNA in vitro: the polymerase chain reaction" Cold Spring Harb Symp Quant Biol. 1986;51 Pt 1:263-73.

## Claims

1. A method for producing lacto-N-triose and/or lacto-N-tetraose and/or lacto-N-neotetraose which comprises carrying out a reaction using at least one culture of genetically modified yeasts which provide the activities of the enzymes responsible for producing lacto-N-triose and/or lacto-N-tetraose and/or lacto-N-neotetraose from lactose, which comprises:
a) adding lactose and N-acetylglucosamine in the presence of beta-(1,3)-N-acetylglucosaminyltransferase to form lacto-N-triose, and
b) adding galactose in the presence of beta-galactosyltransferase to form lacto-N-tetraose and/or lacto-N-neotetraose,
c) allowing lacto-N-triose and/or lacto-N-tetraose and/or lacto-N-neotetraose to accumulate, and
d) recovering lacto-N-triose and/or lacto-N-tetraose and/or lacto-N-neotetraose from said reaction mixture,
**characterized in that**
the enzymes necessary for producing the lacto-N-triose, and/or lacto-N-tetraose and/or lacto-N-neotetraose are produced by genetically modified yeasts from the species *S*. *cerevisiae,* which
carry a recombinant vector comprising the *nagA* gene of *Aspergillus niger* SEQ ID NO: 1 expressing an enzyme with beta-1-3-N-acetylglucosaminyltransferase activity and
a recombinant vector comprising the *lacZ* gene of *Streptococcus thermophilus* SEQ ID NO: 2 expressing an enzyme with beta-galactosyltransferase activity.

2. The method according to claim 1, **characterized in that** a single yeast from the species S. *cerevisiae* is the carrier of one or more vectors comprising the *nagA* gene of *Aspergillus niger* SEQ ID NO: 1 expressing an enzyme with beta-1-3-N-acetylglucosaminyltransferase activity and the *lacZ* gene of *Streptococcus thermophilus* SEQ ID NO: 2 expressing an enzyme with beta-galactosyltransferase activity.

3. The method according to any of the preceding claims, **characterized in that** the enzymatic activity of N-acetylglucosaminyltransferase and/or beta-galactosyltransferase is recovered from the culture broth, cell extract or enzymes purified from the culture medium.

4. The method according to any of the preceding claims, **characterized in that** said step (a) of the method lasts for about 70 to 74 hours at a temperature of 43°C to 47°C.

5. The method according to any of the preceding claims, **characterized in that** said step (a) of the method lasts for about 72 hours at a temperature of 45°C.

6. The method according to any of the preceding claims, **characterized in that** it additionally comprises a step of extracting and purifying lacto-N-triose obtained in step (a).

7. The method according to any of the preceding claims, **characterized in that** the beta-galactosyltransferase enzyme used in step b) is beta-1-3-galactosyltransferase to form lacto-N-tetraose and/or beta-1-4-galactosyltransferase to form lacto-N-neotetraose.

8. The method according to any of the preceding claims, **characterized in that** lacto-N-tetraose and/or lacto-N-neotetraose are obtained from culture broth rich in nagA activity and cell extract with lacZ activity originating from a single strain of yeast from the species *S*. *cerevisiae* expressing and secreting nagA SEQ ID NO: 1 and expressing and not secreting lacZ SEQ ID NO: 2.

9. The method according to any of the preceding claims, **characterized in that** lacto-N-tetraose and/or lacto-N-neotetraose are obtained from culture broth rich in nagA activity and lacZ activity originating from a single strain of yeast from the species S. *cerevisiae* expressing and secreting nagA SEQ ID NO: 1 and expressing and secreting lacZ SEQ ID NO: 2.

## Patentansprüche

1. Verfahren zum Herstellen von Lacto-N-triose und/oder Lacto-N-tetraose und/oder Lacto-N-neotetraose, umfassend das Durchführen einer Reaktion mit mindestens einer Kultur von genetisch veränderten Hefen, die die Aktivitäten der Enzyme bereitstellen, die zum Herstellen von Lacto-N-triose und/oder Lacto-N-tetraose und/oder Lacto-N-neotetraose aus Lactose verantwortlich sind, welches Folgendes umfasst:
a) Zugeben von Lactose und N-Acetylglucosamin in Gegenwart von beta-(1,3)-N-Acetylglucosaminyltransferase, um Lacto-N-triose zu bilden, und
b) Zugeben von Galactose in Gegenwart von beta-Galactosyltransferase, um Lacto-N-tetraose und/oder Lacto-N-neotetraose zu bilden,
c) Ermöglichen einer Akkumulation von Lacto-N-triose und/oder Lacto-N-tetraose und/oder Lacto-N-neotetraose, und
d) Wiedergewinnen von Lacto-N-triose und/oder Lacto-N-tetraose und/oder Lacto-N-neotetraose aus dem Reaktionsgemisch.
**dadurch gekennzeichnet, dass** die Enzyme, die zum Herstellen der Lacto-N-triose und/oder Lacto-N-tetraose und/oder Lacto-N-neotetraose notwendig sind, durch genetisch veränderte Hefen von der Spezies *S*. *cerevisiae* hergestellt sind, welche einen rekombinanten Vektor, umfassend das *nagA*-Gen von *Aspergillus niger* der SEQ ID NO: 1, das ein Enzym mit beta-1-3-N-Acetylglucosaminyltransferase-Aktivität exprimiert, und einen rekombinanten Vektor, umfassend das *lacZ*-Gen von *Streptococcus thermophilus* der SEQ ID NO: 2, das ein Enzym mit beta-Galactosyltransferase-Aktivität exprimiert, tragen.

2. Verfahren nach Anspruch 1, **dadurch gekennzeichnet, dass** eine einzelne Hefe von der Spezies *S*. *cerevisiae* der Träger von einem oder mehreren Vektoren ist, umfassend das *nagA*-Gen von *Aspergillus niger* der SEQ ID NO: 1, das ein Enzym mit beta-1-3-N-Acetylglucosaminyltransferase-Aktivität exprimiert, und das *lacZ*-Gen von *Streptococcus thermophilus* der SEQ ID NO: 2, das ein Enzym mit beta-Galactosyltransferase-Aktivität exprimiert.

3. Verfahren nach einem der vorhergehenden Ansprüche, **dadurch gekennzeichnet, dass** die enzymatische Aktivität von N-Acetylglucosaminyltransferase und/oder beta-Galactosyttransferase von der Kulturbrühe, dem Zellextrakt oder Enzymen, die von dem Kulturmedium gereinigt sind, zurückgewonnen wird.

4. Verfahren nach einem der vorhergehenden Ansprüche, **dadurch gekennzeichnet, dass** der Schritt (a) des Verfahrens etwa 70 bis 74 Stunden bei einer Temperatur von 43 °C bis 47 °C dauert.

5. Verfahren nach einem der vorhergehenden Ansprüche, **dadurch gekennzeichnet, dass** der Schritt (a) des Verfahrens etwa 72 Stunden bei einer Temperatur von 45 °C dauert.

6. Verfahren nach einem der vorhergehenden Ansprüche, **dadurch gekennzeichnet, dass** es zusätzlich einen Schritt zum Extrahieren und Reinigen von Lacto-N-triose, die in Schritt (a) erhalten wurde, umfasst.

7. Verfahren nach einem der vorhergehenden Ansprüche, **dadurch gekennzeichnet, dass** das beta-Galactosyltransferase-Enzym, das in Schritt b) verwendet wurde, beta-1-3-Galactosyltransferase, um Lacto-N-tetraose zu bilden, und/oder beta-1-4-Galactosyltransferase, um Lacto-N-neotetraose zu bilden, ist.

8. Verfahren nach einem der vorhergehenden Ansprüche, **dadurch gekennzeichnet, dass** Lacto-N-tetraose und/oder Lacto-N-neotetraose aus einer Kulturbrühe, die reich an nagA-Aktivität ist, und einem Zellextrakt mit lacZ-Aktivität, der aus einem einzelnen Hefestamm von der Spezies S*. cerevisiae* stammt, der nagA-SEQ ID NO: 1 exprimiert und sezerniert und lacZ-SEQ ID NO: 2 exprimiert und nicht sezerniert, erhalten werden.

9. Verfahren nach einem der vorhergehenden Ansprüche, **dadurch gekennzeichnet, dass** Lacto-N-tetraose und/oder Lacto-N-neotetraose aus einer Kulturbrühe, die reich an nagA-Aktivität und lacZ-Aktivität ist, die aus einem einzelnen Hefestamm von der Spezies *S*. *cerevisiae* stammt, der nagA-SEQ ID NO: 1 exprimiert und sezerniert und lacZ-SEQ ID NO: 2 exprimiert und sezerniert, erhalten wird.

## Revendications

1. Procédé pour la production de lacto-N-triose et/ou lacto-N-tétraose et/ou lacto-N-néotétraose qui comprend la mise en oeuvre d'une réaction en utilisant au moins une culture de levures génétiquement modifiées qui fournissent les activités des enzymes responsables de la production de lacto-N-triose et/ou lacto-N-tétraose et/ou lacto-N-néotétraose à partir de lactose, qui comprend :
a) l'ajout de lactose et de N-acétylglucosamine en présence de béta-(1,3)-N-acétylglucosaminyltransférase pour former le lacto-N-triose, et
b) l'ajout de galactose en présence de béta-galactosyltransférase pour former le lacto-N-tétraose et/ou lacto-N-néotétraose.
c) laisser s'accumuler le lacto-N-triose et/ou lacto-N-tétraose et/ou lacto-N-néotétraose, et
d) la récupération de lacto-N-triose et/ou lacto-N-tétraose et/ou lacto-N-néotétraose dudit mélange de réaction.
**caractérisé en ce que**
les enzymes nécessaires pour produire le lacto-N-triose, et/ou lacto-N-tétraose et/ou lacto-N-néotétraose sont produites par des levures génétiquement modifiées à partir des espèces *S*. *cerevisiae,* qui
portent un vecteur recombinant comprenant le gène *nagA* de *Aspergillus niger* SEQ ID NO: 1 exprimant une enzyme avec une activité béta-1-3-N-acétylglucosaminyltransférase et
un vecteur recombinant comprenant le gène *lacZ* de *Streptococcus thermophilus* SEQ ID NO : 2 exprimant une enzyme avec une activité béta-galactosyltransférase.

2. Procédé selon la revendication 1, **caractérisé en ce qu'**une seule levure provenant des espèces *S*. *cerevisiae* est le porteur d'un ou plusieurs vecteurs comprenant le gène *nagA* de *Aspergillus niger* SEQ ID NO : 1 exprimant une enzyme avec une activité béta-1-3-N-acétylglucosaminyltransférase et le gène *lacZ* de *Streptococcus thermophilus* SEQ ID NO : 2 exprimant une enzyme avec une activité béta-galactosyltransférase.

3. Procédé selon l'une quelconque des revendications précédentes, **caractérisé en ce que** l'activité enzymatique de N-acétylglucosaminyltransférase et/ou béta-galactosyltransférase est récupérée du bouillon de culture, de l'extrait cellulaire ou des enzymes purifiées du milieu de culture.

4. Procédé selon l'une quelconque des revendications précédentes, **caractérisé en ce que** ladite étape (a) du procédé dure pendant 70 à 74 heures environ à une température de 43 °C à 47 °C.

5. Procédé selon l'une quelconque des revendications précédentes, **caractérisé en ce que** ladite étape (a) du procédé dure pendant 72 heures environ à une température de 45 °C.

6. Procédé selon l'une quelconque des revendications précédentes, **caractérisé en ce qu'**il comprend en outre une étape d'extraction et de purification de lacto-N-triose obtenu dans l'étape (a).

7. Procédé selon l'une quelconque des revendications précédentes, **caractérisé en ce que** l'enzyme béta-galactosyltransférase utilisée dans l'étape b) est le béta-1-3- galactosyltransférase pour former le lacto-N-tétraose et/ou béta-1-4 galactosyltransférase pour former le lacto-N-néotétraose.

8. Procédé selon l'une quelconque des revendications précédentes, **caractérisé en ce que** le lacto-N-tétraose et/ou lacto-N-néotétraose sont obtenus du bouillon de culture riche en activité de nagA et en extrait cellulaire avec une activité de lacZ provenant d'une seule souche de levure des espèces *S*. *cerevisiae* exprimant et sécrétant nagA SEQ ID NO : 1 et exprimant et ne sécrétant pas lacZ SEQ ID NO : 2.

9. Procédé selon l'une quelconque des revendications précédentes, **caractérisé en ce que** le lacto-N-tétraose et/ou le lacto-N-néotétraose sont obtenus du bouillon de culture riche en activité de nagA et activité lacZ provenant d'une seule souche de levure des espèces *S*. *cerevisiae* exprimant et sécrétant nagA SEQ ID NO : 1 et exprimant et sécrétant lacZ SEQ ID NO : 2.
